# EUROPÄISCHE PATENTSCHRIFT

(11) **EP 1 725 300 B1**
(45) Veröffentlichungstag und Bekanntmachung des Hinweises auf die Patenterteilung: **21.07.2010**
(21) Anmeldenummer: 05716715.7
(22) Anmeldetag: 16.02.2005
(51) Int. Cl.: A61Q 15/00, A61K 8/44, A61K 8/81, C08F 26/00

(54) **Die Verwendung eines Polymers, Copolymers oder Terpolymers als anti-adhäsiver Wirkstoff gegenüber Mikroorganismen auf der haut.**
The use of a polymer, copolymer or terpolymer as an anti-adhesive active against Microorganisms on the skin.
L'utilisation d'une polymère, copolymère ou terpolymère comme agent d'anti-adhérence des microorganismes sur la peau

(30) Priorität: 11.03.2004 DE 102004012363
(43) Veröffentlichungstag der Anmeldung: 29.11.2006
(73) Patentinhaber: Beiersdorf AG, 20253 Hamburg (DE)
(72) Erfinder: VON THADEN, Stefanie, 20255 Hamburg (DE); WARNKE, Katja, 22547 Hamburg (DE); MEIER-ZIMMERER, Cornelia, 22529 Hamburg (DE); FÖLSTER, Heike, 20257 Hamburg (DE); SCHÄFER, Andreas, 22299 Hamburg (DE)
(86) Internationale Anmeldenummer: PCT/EP2005/050685
(87) Internationale Veröffentlichungsnummer: WO 2005/087188

(56) Entgegenhaltungen:
- EP-A- 0 838 155
- DE-A1- 19 503 423
- DE-A1- 19 841 794
- FR-A- 2 777 184
- US-A1- 2004 047 885
- US-A1- 2004 052 746

## Beschreibung

Die vorliegende Erfindung betrifft ein Polymer, Copolymer oder Terpolymer, welches durch Polymerisation von Monomeren oder Monomermischungen erzeugt wird, enthaltend mindestens ein Monomer, das mit basischen, Heteroatomen enthaltenden Gruppen substituiert ist, wobei es sich bei den basischen Gruppen um basisch substituierte Vinylmonomere handelt, als anti-adhäsiver Wirkstoff gegenüber Mikroorganismen auf der Haut in kosmetische Deodorant und/oder Antitranspirant-Zubereitungen und als anti-adhäsiver Wirkstoff gegenüber Mikroorganismen auf der Haut.

Der gesunde warmblütige Organismus, insbesondere die gesunde menschliche Haut, ist mit einer Vielzahl nicht-pathogener Mikroorganismen besiedelt. Diese sogenannte Mikroflora der Haut ist nicht nur unschädlich, sie stellt einen wichtigen Schutz zur Abwehr opportunistischer oder pathogener Keime dar.

Zu den Mikroorganismen zählen u.a. Bakterien. Bakterien gehören zu den prokaryotischen Einzellern. Sie können grob nach ihrer Form (Kugel, Zylinder, gekrümmter Zylinder) sowie nach dem Aufbau ihrer Zellwand (grampositiv, gramnegativ) unterschieden werden. Feinere Unterteilungen tragen auch der Physiologie der Organismen Rechnung. So existieren aerobe, anaerobe sowie fakultativ anaerobe Bakterien. Manche Individuen sind in ihrer Eigenschaft als pathogene Keime von medizinischer Bedeutung, andere wiederum sind vollkommen harmlos.

Mikroorganismen (insbesondere grampositive Bakterien) spielen bei der Entstehung von Körpergeruch eine entscheidende Rolle. Körpergeruch entsteht bei der Einwirkung von (grampositiven) Hautbakterien auf apokrinen Schweiß, der diesen Mikroorganismen, insbesondere in den feuchten, warmen Achselhöhlen oder unter ähnlichen Bedingungen (z.B. an den Füßen beim Tragen enger Schuhe) idealen Nährboden bietet. Durch die gerade in diesen Bereichen der Haut besonders hohe Anzahl apokriner Schweißdrüsen wird den Bakterien ein gesteigertes Nahrungsangebot zur Verfügung gestellt, wodurch eine übermäßige Vermehrung dieser Keime ermöglicht wird.

Frisch gebildeter Schweiß ist völlig geruchsfrei. Der durch bakteriellen Stoffwechsel erzeugte Schweißgeruch ist personenspezifisch und bei jedem Menschen unterschiedlich ausgeprägt. Durch einfaches Waschen lässt sich bei den meisten Menschen nur eine kurzfristige Reduzierung des Schweißgeruches erzielen. Um den Schweißgeruch über einen längeren Zeitraum zu unterdrücken, ist der Einsatz kosmetischer Zubereitungen unerlässlich.

Um eine Deowirkung zu erreichen werden in der Regel Deowirkstoffe und/oder Antitranspirantien eingesetzt:
Deowirkstoffe dienen dazu, das Wachstum der den Schweißgeruch verursachenden Bakterien zu unterdrücken. Zu diesen keimreduzierenden (bakterioziden) Mitteln zählen beispielsweise Chlorhexidin, Triclosan oder die natürlich vorkommenden Verbindungen wie Farnesol und Phenoxyethanol.

Durch die Verwendung antimikrobieller Deowirkstoffe in kosmetischen Desodorantien kann die Bakterienflora auf der Haut reduziert werden. Dabei sollten im Idealfalle nur die Geruch verursachenden Mikroorganismen wirksam reduziert werden. In der Praxis hat sich aber herausgestellt, dass die gesamte Mikroflora der Haut beeinträchtigt werden kann. Der Schweißfluß selbst wird dadurch nicht beeinflußt, im Idealfalle wird nur die mikrobielle Zersetzung des Schweißes zeitweilig gestoppt.

Antitranspirantien (Adstringentien) dienen dazu, die Schweißabsonderung durch Blockierung der Schweißdrüsenausgänge zu behindem. Verwendung finden hier in aller Regel Aluminium- und Aluminium/Zirkoniumsalze. Die durch diese Verbindungen verringerte Ausscheidung von Schweiß hat nach neuesten Erkenntnissen keine Auswirkung auf den Organismus, da die 'Kühlwirkung' zum großen Teil über das ,Schwitzen' mit Deostoffen unbehandelter Hautpartien (ekkrine Drüsen) erfolgt.

Ferner werden Parfümstoffe zur Überdeckung des Schweißgeruches eingesetzt.

Auch die Kombination von Adstringentien mit antimikrobiell wirksamen Stoffen in ein und derselben Zusammensetzung ist gebräuchlich. Die Nachteile beider Wirkstoffklassen lassen sich auf diesem Wege jedoch nicht vollständig beseitigen.

Ferner wurden in der Literatur die Verwendung von Kohlenhydraten (WO 96/23479) und Sterolen (EP 0838155) als sogenannte Anti-Adhäsiva beschrieben, welche die Anheftung (Adhäsion) von Mikroorganismen auf der Haut reduzieren. Anti-Adhäsiva lösen dabei zum einen bereits an der Oberfläche adhärente Mikroorganismen ab, zum anderen verhindern sie die Neuansiedelung weiterer Mikroorganismen. Sie wirken im Gegensatz zu Deowirkstoffen weder wachstumshemmend (bakteriostatisch) noch keimabtötend (bakterizid).

Anti-mikrobielle Wirkstoffe verhindern, dass Mikroorganismen den Schweiß zersetzen, indem diese in ihrem Wachstum gehemmt oder abgetötet werden. Die Mikroflora auf der Hautoberfläche wird dadurch reduziert.

Auch die DE 19634021, DE 19643585, DE 19718777, DE 195 03423, EP 0995425 und EP 985404 konnten nicht den Weg zur vorliegenden Erfindung weisen.

Desodorantien sollen folgende Bedingungen erfüllen:
1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in übliche kosmetische Formulierungen einarbeiten lassen.

Eine Aufgabe der vorliegenden Erfindung war es, kosmetische Desodorantien zu entwickeln, welche die Mikroflora der Haut weitgehend schonen, die Mikroorganismen aber, die für den Körpergeruch verantwortlich sind, von der Haut abzulösen oder deren Bindung an die Haut zu vermindern. Es sollte sich um eine besonders hautfreundliche Zubereitung handeln.

Weiterhin war es eine Aufgabe der Erfindung, kosmetische Desodorantien zu entwickeln, die sich durch gute Hautverträglichkeit auszeichnen. Auf keinen Fall sollten die desodorierenden Wirkprinzipien sich auf der Haut anreichern.

Eine weitere Aufgabe der vorliegenden Erfindung war es, den Stand der Technik dadurch zu bereichern, dass Substanzen zur Verfügung gestellt werden, welche gegen grampositive und/oder gramnegative Bakterien wirksam sind, ohne dass mit der Anwendung der Substanzen eine unvertretbare Beeinträchtigung der Gesundheit des Anwenders verbunden wäre.

Eine weitere Aufgabe war, kosmetische Desodorantien zu entwickeln, welche mit einer möglichst großen Vielzahl an üblichen kosmetischen Hilfs- und Zusatzstoffen harmonieren, insbesondere mit den gerade in desodorierend oder antitranspirierend wirkenden Formulierungen bedeutenden Parfümbestandteilen.

Eine weitere Aufgabe der Erfindung war, kosmetische Desodorantien zur Verfügung zu stellen, welche über einen längeren Zeitraum, und zwar in der Größenordnung von mindestens einem halben Tag, wirksam sind, ohne dass ihre Wirkung spürbar nachläßt.

Grampositive Bakterien spielen nicht nur bei der Entstehung von Körpergeruch eine Rolle. Sie beeinflussen auch die Entstehung von unreiner Haut und Akne. Bei der unreinen Haut beispielsweise sind neben anderen Einflüssen bakterielle Sekundärinfektionen von ätiologischer Bedeutung. Einer der wichtigsten Mikroorganismen, der in Zusammenhang mit unreiner Haut steht, ist Propionibacterium acnes.

Unreine Haut und/oder Komedonen beeinträchtigen das Wohlbefinden der Betroffenen aber selbst in leichten Fällen. Da praktisch jeder oder jede Jugendliche von unreiner Haut irgendeiner Ausprägung betroffen ist, besteht bei vielen Personen Bedarf, diesem Zustande abzuhelfen.

Eine besondere Aufgabe der vorliegenden Erfindung war es also, einen gegen unreine Haut bzw. Propionibacterium acnes wirksamen Stoff bzw. Stoffkombination zu finden.

Nicht zuletzt war es die Aufgabe der vorliegenden Erfindung eine Deodorant und/oder Antitranspirat-Zubereitung zu entwickeln, welche anti-adhäsiv auf geruchsbildende Mikroorganismen (insbesondere grampositive Bakterien) wirkt. Da es nach dem Stande der Technik bisher nicht möglich war, größere (= wirksame) Mengen an anti-adhäsiv wirkenden Stoffen in alkoholische Zubereitungen (insbesondere versprühbare alkoholische Zubereitungen) stabil einzuarbeiten, war es die Aufgabe der vorliegenden Erfindung, diesen Mangel zu beseitigen.

Zusätzlich war es die Aufgabe der vorliegenden Erfindung, der bekannten emulsionszerstörenden Wirkung von bisherigen Anti-Adhäsiva entgegenzuwirken, um größere (=wirksame) Mengen an anti-adhäsiv wirkenden Stoffen in Emulsionsprodukte (Roll-on, Zerstäuber, Creme) einarbeiten zu können.

Überraschend und selbst für den Fachmann nicht vorhersehbar, werden die Aufgaben erfindungsgemäß gelöst durch
Die Verwendung eines Polymers, Copolymers oder Terpolymers, welches durch Polymerisation von Monomeren oder Monomermischungen erzeugt wird, enthaltend mindestens ein Monomer, das mit basischen, Heteroatomen enthaltenden Gruppen substituiert ist, wobei es sich bei den basischen Gruppen um basisch substituierte Vinylmonomere handelt, als anti-adhäsiver Wirkstoff gegenüber Mikroorganismen auf der Haut in kosmetischen Deodorant und/oder Antitranspirant-Zubereitungen.
- Die Verwendung eines Polymers, Copolymers oder Terpolymers, welches durch Polymerisation von Monomeren oder Monomermischungen erzeugt wird, enthaltend mindestens ein Monomer, das mit basischen, Heteroatomen enthaltenden Gruppen substituiert ist, wobei es sich bei den basischen Gruppen um basisch substituierte Vinylmonomere handelt,
   als anti-adhäsiver Wirkstoff gegenüber Mikroorganismen auf der Haut.
   Die Verwendung eines Polymers, welches gebildet wird aus
   31 - 40 Gew.-% 2-(N, N-dimethylamino)ethylmethacrylat,
   52 - 62 Gew.-% Ethylacrylat,
   2,5 - 5 Gew.-% Beheneth-25 methacrylat,
   2 - 7 Gew.-% eines Ethers aus Allylalkohol und einem Polyethylenglycoi/Polypropylenglycol-Ether mit durchschnittlich 30 Ethylenglycol und durchschnittlich 4 Polypropylenglycol-Einheiten,
   0,05 - 0,2 Gew.-% Triethylenglycoldimethacrylat und
   1,5 - 2,5 Gew.-% 2-Hydroxyethylmethacrylat,
   wobei sich die Gewichtsangaben auf das Gesamtgewicht des Polymers beziehen,
   als anti-adhäsiver Wirkstoff gegenüber Mikroorganismen auf der Haut in kosmetischen Deodorant und/oder Antitranspirant-Zubereitungen.
   als anti-adhäsiver Wirkstoff gegenüber Mikroorganismen auf der Haut in kosmetischen Deodorant und/oder Antitranspirant-Zubereitungen.
- Die Verwendung eines Polymers, welches gebildet wird aus
   31 - 40 Gew.-% 2-(N, N-dimethylamino)ethylmethacrylat,
   52 - 62 Gew.-% Ethylacrylat,
   2,5 - 5 Gew.-% Beheneth-25 methacrylat,
   2 - 7 Gew.-% eines Ethers aus Allylalkohol und einem Polyethylenglycol/Polypropylenglycol-Ether mit durchschnittlich 30 Ethylenglycol und durchschnittlich 4 Polypropylenglycol-Einheiten,
   0,05 - 0,2 Gew.-% Triethylenglycoldimethacrylat und
   1,5 - 2,5 Gew.-% 2-Hydroxyethylmethacrylat,
   wobei sich die Gewichtsangaben auf das Gesamtgewicht des Polymers beziehen,
   als anti-adhäsiver Wirkstoff gegenüber Mikroorganismen auf der Haut.
- Eine kosmetische Deodorant und/oder Antitranspirant-Zubereitung enthaltend ein Polymer, welches gebildet wird aus
   31 - 40 Gew.-% 2-(N, N-dimethylamino)ethylmethacrylat,
   52 - 62 Gew.-% Ethylacrylat,
   2,5 - 5 Gew.-% Beheneth-25 methacrylat,
   2 - 7 Gew.-% eines Ethers aus Allylalkohol und einem PolyethylenglycollPolypropylenglycol-Ether mit durchschnittlich 30 Ethylenglycol und durchschnittlich 4 Polypropylenglycol-Einheiten,
   0,05 - 0,2 Gew.-% Triethylenglycoldimethacrylat und
   1,5 - 2,5 Gew.-% 2-Hydroxyethylmethacrylat,
   wobei sich die Gewichtsangaben auf das Gesamtgewicht des Polymers beziehen.

Dabei ist es erfindungsgemäß bevorzugt, wenn das erfindungsgemäße Polymer gebildet wird aus
34 - 36 Gew.-% 2-(N, N-dimethylamino)ethylmethacrylat,
55 - 57 Gew.-% Ethylacrylat,
2,5 - 3,5 Gew.-% Beheneth-25 methacrylat,
3,5 - 5 Gew.-% eines Ethers aus Allylalkohol und einem Polyethylenglycol/Polypropylenglycol-Ether mit durchschnittlich 30 Ethylenglycol und durchschnittlich 4 Polypropylenglycol-Einheiten, 0,1 - 0,15 Gew.-% Triethylenglycoldimethacrylat und
2 Gew.-% 2-Hydroxyethylmethacrylat,

wobei sich die Gewichtsangaben auf das Gesamtgewicht des Polymers beziehen.

Erfindungsgemäß besonders bevorzugt sind Polymere, die unter der Bezeichnung EX 794 und EX 797 bei der Firma Noveon erhältlich sind.

Die Polymere Materialien können in fest Form oder als Dispersion in Wasser oder Ölen eingesetzt werden. Eine bevorzugte Einsatzform ist die wässrige Polymer-Dispersion, stabilisiert durch Zugabe von ionischen und/oder nichtionischen oberflächenaktiven Verbindungen. Der Polymergehalt dieser Dispersionen liegt typischerweise zwischen 10-40 Gew%.

Überraschenderweise wurde gefunden, dass die erfindungsgemäßen Polymere und Zubereitungen, die sie enthalten, die Adhäsion, d.h. das Vermögen der Mikroorganismen (insbesondere Bakterien) an Oberflächen anzuhaften, herabsetzen, so dass sich deren übliche Anzahl auf solche Flächen verringert, oder auch, dass sich keine oder keine wesentlichen Mengen von Mikroorganismen (insbesondere Bakterien) mehr nachweisen lassen.

Solche Oberflächen sind z.B. Organaußenflächen oder Organ-Oberflächen, insbesondere von der Haut oder Schleimhaut und Körperhöhlen (insbesondere die Achselhöhlen).

Mit den Begriffen santi-adhäsiv" und "anti-adhäsiver Wirkung" der erfindungsgemäßen Polymere ist gemeint, dass die Adhäsion von Mikroorganismen (insbesondere Bakterien) an Oberflächen herabgesetzt oder aufgehoben ist.

Die erfindungsgemäßen anti-adhäsiven Polymere und Zubereitungen können prophylaktisch verwendet werden und bewirken, dass sich auf Oberflächen nur noch geringe und keine störenden Ansammlungen von Mikroorganismen (z.B. Bakterien, insbesondere Schweißgeruch verursachende Bakterien) ausbilden, oder sie verdrängen auch bereits anhaftende Mikroorganismen, Parasiten und Protozoen von der Oberfläche und verringern so deren Anzahl.

Mit den erfindungsgemäßen Polymeren und Zubereitungen, die diese enthalten, kann die Adhäsion von Mikroorganismen an Oberflächen verringert oder vermieden werden. Dies ist beispielsweise der Fall bei Bakterien, und zwar bei grampositiven und bei gramnegativen Bakterien, Hefen, Pilzen und Dermatophyten.

Insbesondere die folgenden Mikroorganismen beispielsweise und die durch sie hervorgerufenen Störungen und Krankheiten können erfindungsgemäß besonders gut, insbesondere topisch behandelt werden:

### 1. Grampositive Bakterien

Als Beispiele:
Fakultativ pathogene und pathogene Micrococcaceae, insbesondere Staphylococcus epidermidis, z.B. bei der Entstehung von Achselgeruch und beim atopischen Ekzem sowie Staphylococcus aureus als wichtiges Pathogen, z.B. beim atopischen Ekzem, bei Neurodermitis und Psoriasis, Corynebacterium spec., z.B. bei der Entstehung des Achselgeruches, Propionibacterium spec., z.B. bei der Entstehung von Akne und bei unreiner Haut.

### 2. Gramnegative Bakterien

Als Beispiele:
Escherichia coli, z.B. bei Colitis ulcerosa; Pseudomonas aeruginosa, z.B. bei Superinfektionen offener Wunden, z.B. im Bauchraum sowie bei cystischer Fibrosis, Enterococcaceae, z.B. bei Magen-Darm-Infektionen. Die Wirkstoffe können bei Spülungen und peroral verwendet werden.

Zubereitungen, die erfindungsgemäße Wirkstoffe enthalten, können erfindungsgemäß bevorzugt topische Zubereitungen sein, beispielsweise kosmetische und dermatologische topische Zubereitungen oder aber auch übliche Arzneimittel-Darreichungsformen. Besonders bevorzugt werden Desodorantien oder desodorierende Körperreinigungsprodukte oder Körperpflegeprodukte. Die Wirkstoffe können aber auch in Desinfektionsmitteln und/oder Reinigungsmitteln enthalten sein, die nicht nur zur Behandlung des Körpers oder der Haut bestimmt sind, sondern auch zum Reinigen und Desinfizieren von harten Oberflächen, medizinischen Materialien, Geräten, Instrumenten, Mobiliar und Wänden.

Die erfindungsgemäßen Zubereitungen und Zubereitungen, in denen die erfindungsgemäßen Verwendungen verwirklicht sind, sind **dadurch gekennzeichnet, dass** das Polymer in der Zubereitung erfindungsgemäß vorteilhaft in einer Konzentration von 0,01-10 Gewichts-% und erfindungsgemäß bevorzugt in einer Konzentration von 0,1-1 Gewichts-%, jeweils bezogen auf das Gesamtgewicht der Zubereitung enthalten ist.

Die wasserlösliche Phase der erfindungsgemäßen Zubereitungen kann neben Wasser erfindungsgemäß auch andere Inhaltsstoffe enthalten, beispielsweise Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäß vorteilhaft lassen sich große Mengen saurer Aluminium- und/oder Aluminium/Zirkoniumsatze stabil in die Zubereitungen einarbeiten. Es können 5 bis 20 Gewichts-%, insbesondere 10 bis 20 Gewichts-% Aluminiumchlomydrat und/oder Aluminium/Zirkoniumchlorhydrat stabil in die Emulsionen eingearbeitet werden. Hierbei beziehen sich die beschriebenen Konzentrationsbereiche auf die sogenannte Aktivgehalte der Antitranspirant-Komplexe: bei den Aluminium-Verbindungen auf wasserfreie Komplexe, bei den Aluminium/Zirkonium-Verbindungen auf wasser- und pufferfreie Komplexe. Als Puffer wird hier üblicherweise Glycin verwendet.

Die nachfolgende Auflastung vorteilhaft einzusetzender Antitranspirant-Wirker soll in keinster Weise einschränkend sein:
Aluminium-Salze (der empirischen Summenformel [AI₂(OH)ₘClₙ], wobei m+n=6):
   - Aluminium-Salze wie Aluminiumchlorid AlCl₃, Aluminiumsulfat Al₂(SO₄)₃
   - Aluminiumchlorhydrat [Al₂(OH)₅Cl] x H₂O
      Standard Al-Komplexe: Locron L (Clariant), Chlorhydrol (Reheis), ACH-303 (Summit), Aloxicoll L (Giulini).
      Aktivierte Al-Komplexe: Reach 501 (Reheis), AACH-324 (Summit)
   - Aluminiumsesquichlorhydrat [Al₂(OH)_{4,5}Cl_{1,5]} x H₂O
      Standard Al-Komplexe: Aluminum Sesquichlorohydrate (Reheis), ACH-308 (Summit), Aloxicoll 31 L (Giulini)
      Aktivierte Al-Komplexe: Reach 301 (Reheis)
   - Aluminiumdichlofiydrat [Al₂(OH)₄Cl₂] x H₂O
Aluminium-Zirkonium-Salze:
   - Aluminium/Zirkonium Trichlorhydrex Glycin [Al₄Zr(OH)₁₃Cl₃] x H₂O x Gly Standard Al/Zr-Komplexe: Rezal 33GP (Reheis), AZG-7164 (Summit), Zirkonal P3G (Giulini)
      Aktivierte Al/Zr-Komplexe: Reach AZZ 902 (Reheis), AAZG-7160 (Summit), Zirkonal AP3G (Giulini)
   - Aluminium/Zirkonium Tetrachlorhydrex Glycin [Al₄Zr(OH)₁₂Cl₄] x H₂O x Gly Standard Al/Zr-Komplexe: Rezat 36G (Reheis), AZG-368 (Summit), Zirkonal L435G (Giulini)
      Aktivierte Al/Zr-Komplexe: Reach AZP 855 (Reheis), AAZG-6313-15 (Summit), Zirkonal AP4G (Giulini)
   - Atuminium/Zirkonium Pentachlorhydrex Glycin [Al₈Zr(OH)₂₃Cl₅] x H₂O x Gly Standard Al/Zr-Komplexe: Rezal 67 (Reheis), Zirkonal L540 (Giulini)
      Aktivierte Al/Zr-Komplexe: Reach AZN 885 (Reheis)
   - A)uminium/Zirkonium Octachlorhydrex Glycin [Al₈Zr(OH)₂₀Cl₈] x H₂O x Gly
      Ebenso von Vorteil können aber auch Glycin-freie Aluminium/Zirkonium-Salze sein.

Dabei soll die Verwendung der Antitranspirant-Wirker aus den Rohstoffklassen Aluminium- und Atuminium/Zirkonium-Satzen nicht auf die handelsüblichen zumeist wäßrigen Lösungen, wie z.B. Locron L (Clariant), beschränkt sein, sondern es kann auch von Vorteil sein, die ebenfalls handelsüblichen wasserfreien Pulver derselbigen Rohstoffe durch Einbringung in die beanspruchten Formulierungen zum Einsatz zu bringen, wie z.B. Locron P (Clariant).

Vorteilhaft kann auch die Verwendung von sogenannten Antitranspirant-Salz Suspensionen sein, bei denen pulverförmig vorliegende Aluminium- und Aluminium/Zirkonium-Salze in diversen Ölen dispergiert angeboten werden.

Desweiteren kann es aber auch von Vorteil sein, spezielle Aluminium- und Aluminium/Zirkonium-Satze zum Einsatz zu bringen, die zur Löslichkeitsverbesserung als Glykol-Komplexe angeboten werden.

Weitere vorteilhafte Antitranspirant-Wirker basieren anstelle von Aluminium bzw. Zirkonium auf anderen Metallen, wie z.B. Beryllium, Titan, Hafnium.

Dabei soll die Liste der verwendbaren Antitranspirant-Wirker aber nicht auf metallhaltige Rohstoffe begrenzt sein, sondem von Vorteil sind auch Verbindungen, die Nichtmetalle wie Bor enthalten sowie solche, die dem Bereich der organischen Chemie zuzurechnen sind, wie z.B. Anticholinergika.

Der in zahlreichen Zubereitungen auftretende Effekt, dass nach dem Auftragen der Zubereitung auf der Haut ein sichtbarer weißlicher Rückstand zurückbleibt, wird in der Regel vom Anwender als störend empfunden. In wasserfreien Zubereitungen hat sich der Einsatz von propoxylierten Alkoholen zur Kaschierung dieser Erscheinung bewährt. Im Falle von wasserhaltigen Zubereitungen ist bisher keine zufriedenstellende Lösung dieses Problems bekannt. Der Zusatz von propoxylierten Alkoholen mit 10 bis 20 Propyloxyeinheiten und 2 bis 10 Kohlenstoffatomen in der Alkylkette, insbesondere PPG-14-Butylether, als Bestandteil der Lipidphase hilft dem beschriebenen Mangel des Standes der Technik ab, indem das Aufireten derartiger weißlicher Rückstände zuverlässig kaschiert wird.

Vorteilhaft können erfindungsgemäßen Zubereitungen Desodorantien zugesetzt werden. Den üblichen kosmetischen Desodorantien liegen unterschiedliche Wirkprinzipien zugrunde.

Alle für Desodorantien gängigen Wirkstoffe können vorteilhaft genutzt werden, beispielsweise Geruchsüberdecker wie die gängigen Parfümbestandteile, Geruchsabsorber, beispielsweise die in der DE 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen Emulsionen eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Famesol (3,7,11-Trimethyl-2,6,10-dodecatrien-1-ol) sowie die in den DE 37 40 186, DE 39 38 140, DE 42 04 321, DE 42 29 707, DE 42 29 737, DE 42 37 081, DE 43 09 372, DE 43 24 219 beschriebenen wirksamen Agenzien. Auch Natriumhydrogencarbonat ist vorteilhaft zu verwenden.

Die Liste der genannten Wirkstoffe bzw. Wirkstoffkombinationen, die in den erfindungsgemäßen Emulsionen verwendet werden können, soll selbstverständlich nicht limitierend sein.

Die Menge der Desodorantien (eine oder mehrere Verbindungen) in den Zubereitungen beträgt vorzugsweise 0,01 bis 10 Gew.%, besonders bevorzugt 0,05 bis 5 Gew.%, insbesondere 0,1 bis 1 Gew.%, bezogen auf das Gesamtgewicht der Zubereitung.

Die kosmetischen und dermatologischen Zubereitungen können erfindungsgemäß kosmetische Hilfsstoffe enthalten, wie sie üblicherweise in solchen Zubereitungen verwendet werden, z.B. Konservierungsmittel, Antioxidanien, Bakterizide, Parfüme, Emulgatoren, weichmachende, anfeuchtende und/oder feuchthaltende Substanzen, Fette, Öle, Wachse, Farbstoffe, Pigmente, die eine färbende Wirkung haben, Verdickungsmittel oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate. Auch die Einarbeitung von UV-Lichtschutzfiltem ist erfindungsgemäß vorteilhaft. Die aufgeführte Liste an Zusatzstoffen soll selbstverständlich nicht limitierend sein.

Die erfindungsgemäßen Zubereitungen und Zubereitungen, in denen die erfindungsgemäßen Verwendungen verwirklicht sind, können in Form einer Öl-in-Wasser-Emulsion, einer wässrigen Lösung, einer wässrig-alkoholischen Lösung, einer alkoholische Lösung, einem Gel oder Stift vorliegen.

Erfindungsgemäß ist ferner ein Kosmetikum aus einem Sprühapplikator und einer erfindungsgemäßen Zubereitung, wobei der Sprühapplikator erfindungsgemäß in Form eines Pumpzerstäubers oder einer Aerosoldose vorliegt.

Die erfindungsgemäße Zubereitung kann in einem solchen Falle erfindungsgemäß vorteilhaft mit Hilfe eines Treibgases aus dem Vorratsbehältnis ausgetragen werden, wobei als Treibgase erfindungsgemäß bevorzugt Propan, Isobutan und n-Butan sowie deren Mischungen eingesetzt werden.

Das Treibgas wird erfindungsgemäß in einer Menge von 0,5 bis 60 Gewichts-%, besonders vorteilhaft in einer Menge von 5 bis 50 und ganz besonders vorteilhaft in einer Menge von 6 bis 40 Gewichts-%, jeweils bezogen auf das Gesamtgewicht der Formulierung eingesetzt.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Die überraschenden anti-adhäsiven Effekte der vorliegenden Erfindung ließen sich beispielsweise mit dem folgenden Vergleichsversuch belegen:
Bakterien einer ÜNK (Übemacht-Kultur) werden abzentrifugiert, mit Puffer gewaschen, 30 min in 70% EtOH fixiert und mit Propidiumiodid gefärbt. Der überschüssige Farbstoff wird durch mehrmaliges Waschen entfernt. Die Bakterien werden für 1 Std. mit den Rohstoffen (Anti-Adhäsiva) inkubiert. Anschließend werden die Bakteriensuspensionen mit humanen Comeocyten, die vom Unterarm eines Probanden gewonnen werden, 1 Std. bei RT inkubiert. Nach dieser Adhäsionsperiode werden die Bakterien-Comeocyten-Komplexe durch Abtrennung der nicht-adhärenten Bakterien gewonnen und die Adhäsionsrate durchflusszytometrisch bestimmt und mikroskopisch kontrolliert. Die Adhäsionsrate des Kontrollansatzes, in dem Bakterien (nicht vorbehandelt) und Comeozyten inkubiert werden, wird als 100% realtive Adhäsionsrate definiert. Alle anderen Ansätze werden in Relation dazu berechnet. Werte < 100% zeigen eine Inhibition der bakteriellen Adhäsion an, Werte >100 % eine verstärkte bakterielle Adhäsion an.

In Grafik 1 sind die relativen Adhäsionsraten nach Inkubation mit verschiedenen kationischen Polymeren dargestellt. Nicht alle getesteten Polymere zeigen eine inhibitorische Wirkung auf die bakterielle Adhäsion an humane Corneocyten. Hydroxypropyl-Guar (0,1%) hatte keinen Einfluss auf die bakterielle Adhäsion, einen geringen Effekt zeigten Polyquaternium 10 /2254 (0,1%) und Polyquaternium 10/10198 (0,1%), die die Adhäsionsrate um 12,1 bzw. 13,9 % verringerten.

Beide Beispielpolymere zeigen eine sehr gute Reduktion der bakteriellen Adhäsion. EX 794 reduzierte die Adhäsion um 82% (18% relative Adhäsionsrate) und EX 797 verringerte die Adhäsion um 79% (21% relative Adhäsionsrate).

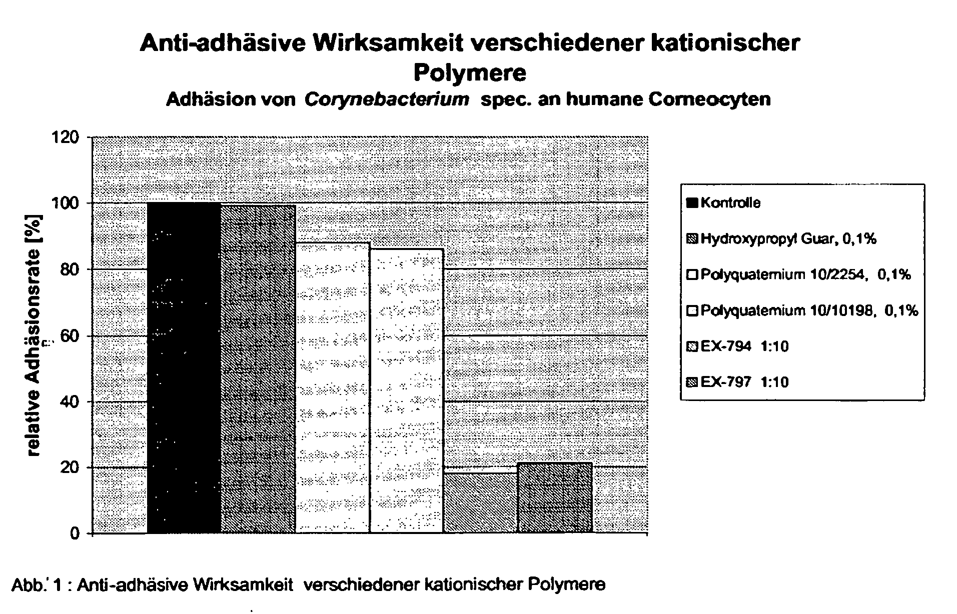

Die mangelnde antimikrobielle Deo-Wirkung der erfindungsgemäßen Polymere ließ sich mit Hilfe des folgenden Vergleichsversuches belegen:
Bakterien einer Übemachtkultur werden mit den zu testenden Substanzen für 3 Std. inkubiert, wobei auch als Kontrolle Bakterien nur mit Medium mitgeführt werden. Nach 20, 60 und 180 Min. werden aus diesen Proben Aliquots in geeigneter Verdünnungsstufe auf Nährmedien ausplatiert, die 48 Std. inkubiert werden. Die gewachsenen Kolonien werden anschließend ausgezählt. Durch den Vergleich mit der Kontrolle können die Rohstoffe dann auf ihre antimikrobielle Wirkleistung beurteilt werden.

**Tabelle : Anti-mikrobielle Wirksamkeit kationisch derivatisierter Acrylate auf Corynebacterium spec.**

| | cfu/ml | cfu/ml | cfu/ml | cfu/ml |
|---|---|---|---|---|
| Zeit [min] | 0 | 20 | 60 | 180 |
| Kontrolle | 2,65E+06 | 3,17E+06 | 5,29E+06 | 1,36E+07 |
| Ex 797 1:10 | 2,65E+06 | 2,92E+06 | 4,66E+06 | 7,49E+06 |
| Ex 794 1:10 | 2,65E+06 | 3,13E+06 | 5,13E+06 | 9,74E+06 |

Die Tabelle verdeutlicht, dass beide erfindungsgemäßen Beispielpolymere (EX 797 und EX 794) keine antimikrobielle Wirksamkeit besitzen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispielrezepturen

| **Roll-on (opaque Makroemulsion)** | |
|---|---|
| Polyethylenglykol(21)stearylether | 3,00 |
| Polyethylenglykol(2)stearylether | 4,00 |
| Polypropylenglykol(15)stearylether | 4,00 |
| Polyethylenglykol(400) | 1,00 |
| Aluminiumchlorhydrat (50% wäßr. Lösung) | 10,00 |
| Erfindungsgemäßes Polymer* | 1,00 |
| Trinatriumethylendiamintetraaoetat | 1,50 |
| (20% wäßr. Lösung) | |
| Parfüm, Antioxidantien | q.s. |
| Wasser, ad | 100,00 |

| **Roll-on (klares Nanoemulsionsgel)** | |
|---|---|
| Glycerinmonoisostearat | 3,00 |
| Polyoxyethylen(20)isohexadecylether | 6,00 |
| Di-n-Octylether | 7,00 |
| Polyethylenglykol(150)distearat | 1,50 |
| Butylenglykol | 3,00 |
| Aluminiumchlorhydrat (50% wäßr. Lösung) | 20,00 |
| Erfindungsgemäßes Polymer* | 1,00 |
| Jojobaöl | 1,00 |
| Parfüm | 1,00 |
| Wasser, ad | 100,00 |

| **Roll-on (Suspension)** | |
|---|---|
| Decamethylcyclopentasiloxan | 70,00 |
| Quaternium-18 Bentonit | 5,00 |
| Propylencarbonat | 2,00 |
| Aluminiumchlorhydrat | 20,00 |
| Erfindungsgemäßes Polymer* | 1,00 |
| Parfüm | 2,00 |

| **Pumpzerstäuber (transluzente Mikroemulsion)** | |
|---|---|
| Polyoxyethylen(20)cetytstearylether | 3,00 |
| Polyoxyethylen(12)oetylstearylether | 0,50 |
| Glycerinstearat | 3,00 |
| Cetylstearylalkohol | 0,50 |
| Cetylpalmitat | 0,50 |
| Capryl-Caprinsäureester | 5,00 |
| Di-n-Octylether | 5,00 |
| Glycerin | 4,00 |
| Aluminiumchtofiydrat (50% wäßr. Lösung) | 16,00 |
| Erfindungsgemäßes Polymer * | 1,00 |
| Avocadoöl | 1,00 |
| Parfüm | 1,00 |
| Wasser, ad | 100,00 |

| **Pumpzerstäuber (klare Nanoemulsion)** | |
|---|---|
| Glycerinmonoisostearat | 5,00 |
| Polyoxyethylen-20-isohexadecylether | 4,00 |
| Di-n-Octylether | 4,50 |
| Butylenglykol | 2,00 |
| Aluminiumchlorhydrat (50% wäßr. Lösung) | 26,00 |
| Erfindungsgemäßes Polymer* | 1,00 |
| Octyldodecanol | 1,00 |
| Parfüm | 1,00 |
| Wasser, ad | 100,00 |

| **Deo Creme (opaque Makroemulsion)** | |
|---|---|
| Polyethylenglykol(2000)monostearat | 4,50 |
| Glycerinmonostearat | 4,00 |
| Cetylalkohol | 5,00 |
| Decamethylcyclopentasiloxan | 6,00 |
| Paraffinöl | 4,50 |
| Aluminiumchlorhydrat (50% wäßr. Lösung) | 30,00 |
| Erfindungsgemäßes Polymer * | 1,00 |
| Glycerin | 5,00 |
| Octyldodecanol | 1,00 |
| Parfüm | 1,00 |
| Wasser, ad | 100,00 |

| **Aerosolspray Typ A:** | |
|---|---|
| 2-Octyldodecanol | 0,50 |
| 1,2-Propylenglycol | 1,00 |
| Erfindungsgemäßes Polymer* | 1,00 |
| Parfüm | q.s. |
| Ethanol, ad | 100,00 |

Die durch Zusammenmischung der jeweiligen Bestandteile erhaltene flüssige Phase wird mit einem Propan-Butan-Gemisch (2:7) im Verhältnis 39:61 in Aerosolbehälter abgefüllt.

| **Aerosolspray Typ B:** | |
|---|---|
| Aluminiumchlorohydrat | 45,00 |
| Isopropylpalmitat | 25,00 |
| Cyclomethicone, ad | 100,00 |
| Erfindungsgemäßes Polymer* | 1,00 |
| Parfüm | q.s |
| Ethanol, ad. | 100,00 |

Die durch Zusammenmischung der jeweiligen Bestandteile erhaltene flüssige Phase wird mit einem Propan-Butan-Gemisch (2:7) im Verhältnis 17:83 in Aerosolbehälter abgefüllt.

| **Aerosolspray Typ C:** | |
|---|---|
| Aluminiumchlorohydrat | 45,00 |
| Isopropylpalmitat | 25,00 |
| Cyclomethicone | 0,30 |
| Isoparafin, ad | 100,00 |
| Talkum | 10,00 |
| Erfindungsgemäßes Polymer * | 1,00 |
| Parfüm | q.s. |
| Ethanol, ad | 100,00 |

Die durch Zusammenmischung der jeweiligen Bestandteile erhaltene flüssige Phase wird mit einem Propan-Butan-Gemisch (2:7) im Verhältnis 17:83 in Aerosolbehälter abgefüllt.

| **Pumpzerstäuber:** | |
|---|---|
| Ethanol | 55,00 |
| PEG-40 Hydriertes Rizinusöl | 2,00 |
| Glycerin | 1,00 |
| Erfindungsgemäßes Polymer* | 1,00 |
| Parfüm | q.s. |
| Wasser, ad | 100,00 |

| **Roll-on Gel Typ A:** | |
|---|---|
| Ethanol | 50,00 |
| PEG-40 Hydriertes Rizinusöl | 2,00 |
| Hydroxyethylcellulose | 0,50 |
| Erfindungsgemäßes Polymer * | 1,00 |
| Parfüm | q.s. |
| Wasser, ad | 100,00 |

| **Roll-on Gel Typ B:** | |
|---|---|
| Ethanol | 50,00 |
| PEG-40 Hydriertes Rizinusöl | 2,00 |
| Hydroxyethylcellulose | 0,50 |
| Aluminiumchlorohydrat | 10,00 |
| Erfindungsgemäßes Polymer* | 1,00 |
| Parfüm | q.s |
| Wasser, ad | 100,00 |

| **Antitranspirant Stift:** | |
|---|---|
| Stearylalkohol | 25,00 |
| Hydriertes Rizinusöl | 2,00 |
| Cyclomethicone, ad | 100,00 |
| Aluminiumchlorohydrat, Pulver | 20,00 |
| Erfindungsgemäßes Polymer* | 1,00 |
| Parfüm | q.s. |

| **Tränkungsmedium für Tücher:** | |
|---|---|
| Ethanol | 10,00 |
| PEG-40 Hydriertes Rizinusöl | 2,50 |
| Glycerin | 1,00 |
| Erfindungsgemäßes Polymer * | 1,00 |
| Parfüm | q.s. |
| Wasser, ad | 100,00 |

Grundsätzlich eignen sich alle organischen und anorganischen Faserstoffe auf natürlicher und synthetischer Basis als Ausgangsmaterialien für gewebte und nicht gewebte Trägermaterialien (TücherNliese). Besonders geeignet sind Fasern aus 100% Viskose, aber auch aus anderen hochfesten Polymeren wie Polyamid, Polyester und/oder hochgerecktem Polyethylen. Auch Mischungen der genannten Faserarten in verschiedenen Massenvefiäftnissen stellen vorteilhafte Materialen für Tücher dar.

## Patentansprüche

1. Verwendung eines Polymers, Copolymers oder Terpolymers, welches durch Polymerisation von Monomeren oder Monomermischungen erzeugt wird, enthaltend mindestens ein Monomer, das mit basischen, Heteroatome enthaltenden Gruppen substituiert ist, wobei es sich bei den basischen Gruppen um basisch substituierte Vinylmonomere handelt, als anti-adhäsiver Wirkstoff gegenüber Mikroorganismen auf der Haut in kosmetischen Deodorant und/oder Antitranspirant-Zubereitungen.

2. Verwendung eines Polymers, Copolymers oder Terpolymers, welches durch Polymerisation von Monomeren oder Monomermischungen erzeugt wird, enthaltend mindestens ein Monomer, das mit basischen, Heteroatome enthaltenden Gruppen substituiert ist, wobei es sich bei den basischen Gruppen um basisch substituierte Vinylmonomere handelt, als anti-adhäsiver Wirkstoff gegenüber Mikroorganismen auf der Haut.

3. Verwendung eines Polymers, welches gebildet wird aus
31 - 40 Gew.-% 2-(N, N-dimethylamino)ethylmethacrylat,
52 - 62 Gew.-% Ethylacrylat,
2,5 - 5 Gew.-% Beheneth-25 methacrylat,
2 - 7 Gew.-% eines Ethers aus Allylalkohol und einem Polyethylenglycol/Polypropylenglycol-Ether mit durchschnittlich 30 Ethylenglycol und durchschnittlich 4 Polypropylenglycol-Einheiten,
0,05 - 0,2 Gew.-% Triethylenglycoldimethacrylat und
1,5 - 2,5 Gew.-% 2-Hydroxyethylmethacrylat,
wobei sich die Gewichtsangaben auf das Gesamtgewicht des Polymers beziehen,
als anti-adhäsiver Wirkstoff gegenüber Mikroorganismen auf der Haut in kosmetische Deodorant und/oder Antitranspirant-Zubereitungen nach Anspruch 1.

4. Verwendung eines Polymers, welches gebildet wird aus
31 - 40 Gew.-% 2-(N, N-dimethylamino)ethylmethacrylat,
52 - 62 Gew.-% Ethylacrylat,
2,5 - 5 Gew.-% Beheneth-25 methacrylat,
2 - 7 Gew.-% eines Ethers aus Allylalkohol und einem Polyethylenglycol/Polypropylenglycol-Ether mit durchschnittlich 30 Ethylenglycol und durchschnittlich 4 Polypropylenglycol-Einheiten,
0,05 - 0,2 Gew.-% Triethylenglycoldimethacrylat und
1,5 - 2,5 Gew.-% 2-Hydroxyethylmethacrylat,
wobei sich die Gewichtsangaben auf das Gesamtgewicht des Polymers beziehen,
als anti-adhäsiver Wirkstoff gegenüber Mikroorganismen auf der Haut nach Anspruch 2.

5. Kosmetische Deodorant und/oder Antitranspirant-Zubereitung enthaltend ein Polymer, welches gebildet wird aus
31 - 40 Gew.-% 2-(N, N-dimethylamino)ethylmethacrylat,
52 - 62 Gew.-% Ethylacrylat,
2,5 - 5 Gew.-% Beheneth-25 methacrylat,
2 - 7 Gew.-% eines Ethers aus Allylalkohol und einem Polyethylenglycol/Polypropylenglycol-Ether mit durchschnittlich 30 Ethylenglycol und durchschnittlich 4 Polypropylenglycol-Einheiten,
0,05 - 0,2 Gew.-% Triethylenglycoldimethacrylat und
1,5 - 2,5 Gew.-% 2-Hydroxyethylmethacrylat,
wobei sich die Gewichtsangaben auf das Gesamtgewicht des Polymers beziehen.

6. Verwendung nach einem der vorhergehenden Ansprüche oder Zubereitung nach Anspruch 5, **dadurch gekennzeichnet, dass** das Polymer in der Zubereitung in einer Konzentration von 0,01 - 10 Gew.-%, bezogen auf das Gesamtgewicht der Zubereitung enthalten ist.

7. Verwendung oder Zubereitung nach einem der vorhergehenden Ansprüche, **dadurch gekennzeichnet, dass** es sich um eine alkoholische Zubereitung handelt.

8. Kosmetikum aus einem Sprühapplikator und einer Zubereitung nach Anspruch 5.

9. Kosmetikum nach Anspruch 8, **dadurch gekennzeichnet, dass** der Sprühapplikator in Form eines Pumpzerstäubers oder einer Aerosoldose vorliegt.

## Claims

1. Use of a polymer, copolymer or terpolymer which is produced by polymerization of monomers or monomer mixtures, comprising at least one monomer which is substituted with basic groups containing hetero atoms, where the basic groups are basically substituted vinyl monomers, as anti-adhesive active against microorganisms on the skin in cosmetic deodorant and/or antiperspirant preparations.

2. Use of a polymer, copolymer or terpolymer which is produced by polymerization of monomers or monomer mixtures, comprising at least one monomer which is substituted with basic groups containing hetero atoms, where the basic groups are basically substituted vinyl monomers, as anti-adhesive active against microorganisms on the skin.

3. Use of a polymer which is formed from
31-40% by weight of 2-(N,N-dimethylamino)ethyl methacrylate,
52-62% by weight of ethyl acrylate,
2.5-5% by weight of beheneth-25 methacrylate,
2-7% by weight of an ether of allyl alcohol and a polyethylene glycol/polypropylene glycol ether having on average 30 ethylene glycol and on average 4 polypropylene glycol units,
0.05-0.2% by weight of triethylene glycol dimethacrylate and
1.5-2.5% by weight of 2-hydroxyethyl methacrylate, where the weight data refer to the total weight of the polymer,
as anti-adhesive active against microorganisms on the skin in cosmetic deodorant and/or antiperspirant preparations according to Claim 1.

4. Use of a polymer which is formed from 31-40% by weight of 2-(N,N-dimethylamino)ethyl methacrylate,
52-62% by weight of ethyl acrylate,
2.5-5% by weight of beheneth-25 methacrylate,
2-7% by weight of an ether of allyl alcohol and a polyethylene glycol/polypropylene glycol ether having on average 30 ethylene glycol and on average 4 polypropylene glycol units,
0.05-0.2% by weight of triethylene glycol dimethacrylate and
1.5-2.5% by weight of 2-hydroxyethyl methacrylate, where the weight data refer to the total weight of the polymer,
as anti-adhesive active against microorganisms on the skin according to Claim 2.

5. Cosmetic deodorant and/or antiperspirant preparation comprising a polymer which is formed from
31-40% by weight of 2-(N,N-dimethylamino)ethyl methacrylate,
52-62% by weight of ethyl acrylate,
2.5-5% by weight of beheneth-25 methacrylate,
2-7% by weight of an ether of allyl alcohol and a polyethylene glycol/polypropylene glycol ether having on average 30 ethylene glycol and on average 4 polypropylene glycol units,
0.05-0.2% by weight of triethylene glycol dimethacrylate and
1.5-2.5% by weight of 2-hydroxyethyl methacrylate, where the weight data are based on the total weight of the polymer.

6. Use according to one of the preceding claims or preparation according to Claim 5, **characterized in that** the polymer in the preparation is present in a concentration of 0.01-10% by weight, based on the total weight of the preparation.

7. Use or preparation according to one of the preceding claims, **characterized in that** it is an alcoholic preparation.

8. Cosmetic product comprising a spray applicator and a preparation according to Claim 5.

9. Cosmetic product according to Claim 8, **characterized in that** the spray applicator is in the form of a pump atomizer or an aerosol can.

## Revendications

1. Utilisation d'un polymère, copolymère ou terpolymère, qui est produit par polymérisation de monomères ou de mélanges de monomères, contenant au moins un monomère qui est substitué par des groupes basiques contenant des hétéroatomes, les groupes basiques consistant en des monomères vinyliques à substitution basique, en tant que substance active antiadhésive vis-à-vis de micro-organismes sur la peau dans des préparations cosmétiques de déodorants et/ou d'antisudoraux.

2. Utilisation d'un polymère, copolymère ou terpolymère, qui est produit par polymérisation de monomères ou de mélanges de monomères, contenant au moins un monomère qui est substitué par des groupes basiques contenant des hétéroatomes, les groupes basiques consistant en des monomères vinyliques à substitution basique, en tant que substance active antiadhésive vis-à-vis de micro-organismes sur la peau.

3. Utilisation d'un polymère qui est constitué de 31 - 40 % en poids de méthacrylate de 2-(N,N-dimêthylamino)éthyle,
52 - 62 % en poids d'acrylate d'éthyle,
2,5 - 5 en poids de béhéneth-25 méthacrylate,
2 - 7 % en poids d'un éther, à base d'alcool allylique et d'un éther de polyéthylèneglycol/polypropylèneglycol comportant en moyenne 30 groupes éthylèneglycol et en moyenne 4 groupes polypropylèneglycol,
0,05 - 0,2 % en poids de diméthacrylate de triéthylèneglycol et
1,5 - 2,5 % en poids de méthacrylate de 2-hydroxy-éthyle,
les données pondérales se rapportant au poids total du polymère,
en tant que substance active antiadhésive vis-à-vis de micro-organismes sur la peau dans des préparations cosmétiques de déodorants et/ou d'antisudoraux selon la revendication 1.

4. Utilisation d'un polymère qui est constitué de 31 - 40 % en poids de méthacrylate de 2-(N,N-diméthylamina)éthyle,
52 - 62 en poids d'acrylate d'éthyle,
2,5 - 5 % en poids de béhéneth-25 méthacrylate,
2 - 7 % en poids d'un éther, à base d'alcool allylique et d'un éther de polyéthylèneglycol/polypropylèneglycol comportant en moyenne 30 groupes éthylèneglycol et en moyenne 4 groupes polypropylèneglycol,
0,05 - 0,2 % en poids de diméthacrylate de triéthylèneglycol et
1,5 - 2,5 % en poids de méthacrylate de 2-hydroxy-éthyle,
les données pondérales se rapportant au poids total du polymère,
en tant que substance active antiadhésive vis-à-vis de micro-organismes sur la peau selon la revendication 2.

5. Préparation cosmétique de déodorant et/ou d'antisudoral, contenant un polymère qui est constitué de
31 - 40 % en poids de méthacrylate de 2-(N,N-diméthylamino)éthyle,
52 - 62 en poids d'acrylate d'éthyle,
2,5 - 5 en poids de béhéneth-25 méthacrylate,
2 - 7 en poids d'un éther, à base d'alcool allylique et d'un éther de polyéthylèneglycol/polypropylèneglycol comportant en moyenne 30 groupes éthylèneglycol et en moyenne 4 groupes polypropylèneglycol,
0,05 - 0,2 % en poids de diméthacrylate de triéthylèneglycol et
1,5 - 2,5 % en poids de méthacrylate de 2-hydroxy-éthyle,
les données pondérales se rapportant au poids total du polymère.

6. Utilisation selon l'une quelconque des revendications précédentes ou préparation selon la revendication 5, **caractérisée en ce que** le polymère est contenu dans la préparation à une concentration de 0,01 - 10 % en poids, par rapport au poids total de la préparation.

7. Utilisation ou préparation selon l'une quelconque des revendications précédentes, **caractérisée en ce qu'**il s'agit d'une préparation alcoolique.

8. Cosmétique composé d'un applicateur par pulvérisation et d'une préparation selon la revendication 5.

9. Cosmétique selon la revendication 8, **caractérisé en ce que** l'applicateur par pulvérisation se trouve sous forme d'un atomiseur à pompe ou d'une bombe aérosol.
